# EUROPÄISCHE PATENTSCHRIFT

(11) **EP 0 955 041 B1**
(45) Veröffentlichungstag und Bekanntmachung des Hinweises auf die Patenterteilung: **12.11.2003**
(21) Anmeldenummer: 99103751.6
(22) Anmeldetag: 26.02.1999
(51) Int. Cl.: A61K 9/28, A61K 9/20, A61K 9/22

(54) **Wässrige Dispersion geeignet zur Herstellung von Überzugs- und Bindemitteln für feste orale Arzneiformen**
Aqueous dispersion useful for the production of coating and binding means for oral solid medicament
Dispersion aqueuse utile pour la production de milieux d'enrobage ou d'agents liants pour des médicaments solides pour la voie orale

(30) Priorität: 06.03.1998 DE 19809719
(43) Veröffentlichungstag der Anmeldung: 10.11.1999
(73) Patentinhaber: Röhm GmbH & Co. KG, 64293 Darmstadt (DE)
(72) Erfinder: Petereit, Hans-Ulrich, 64291 Darmstadt (DE); Gölz, Silke, 64625 Bensheim (DE); Weisbrod, Wolfgang, 64331 Weiterstadt (DE)

(56) Entgegenhaltungen:
- EP-A- 0 519 870
- WO-A-96/35413
- WO-A-97/41839
- US-A- 5 366 755
- INTERNATIONAL JOURNAL OF PHARMACEUTICS, Bd. 68, 1991, Seiten R1-R3, XP000973793

## Beschreibung

Die Erfindung betrifft eine wäßrige Dispersion, geeignet zur Herstellung von Bindemitteln und Überzügen für feste orale Arzneiformen, enthaltend als Feststoffanteil eine Polymermischung A) aus einem Polymethacrylat-Copolymer und einem Alkalisalz der Carboxymethylcellulose und B) üblichen Zuschlagstoffen. Die Erfindung betrifft weiterhin die Verwendungen der Dispersion.

### Stand der Technik

Wasserlösliche nichtionische Polysaccharidderivate z. B. Hydroxypropylmethylcellulose (HPMC) werden routinemäßig zum Granulieren von Tablettiermischungen und zum einfachen Überziehen von festen Arzneiformen eingesetzt. Seltener verwendet werden lösliche ionische Polysaccharide z B. Natrium-Carboxymethylcellulose (Na-CMC). Beide Polymere bilden jedoch Schichten, die sich in Wasser schnell auflösen und eine hohe Permeabilität besitzen. Daher ist die Schutz- und Isolierwirkung in Arzneiformen begrenzt. Das Bindevermögen für Pigmente ist ebenfalls begrenzt, so daß die Deckkraft von Überzügen für stark gefärbte Kerne nicht ausreicht. Weiterhin neigen die hydrophilen, stark quellenden Polymere beim Anrühren in Wasser zum Verklumpen, was durch spezielle Löseverfahren vermieden werden muß.

DE 40 21 678 A1 beschreibt ein Verfahren zur Herstellung klein dimensionierter Formkörper mit Etofibrat-Gehalt und kontrollierter Wirkstoff-Freisetzung durch Mischung des Wirkstoffs mit einem in Wasser unlöslichem und einem in Wasser löslichen oder quellbaren, physiologisch indifferenten Kolloid und anschließende Extrusion. Die wasserunlöslichen und wasserlöslichen/quellbaren Kolloide können in einem Verhältnis von 1 : 10 bis 90 : 1 verwendet werden. Etofibrat kann z. B. zusammen mit einem Polymethacrylsäureester mit quaternären Ammoniumgruppen (EUDRAGIT® RS) und Na-Carboxymethylcellulose im Verhältnis 2 · 1 abgemischt werden. Zielsetzung ist es, eine möglichst konstante Freisetzung über einen Zeitraum von 4 - 6 Stunden zu erreichen.

EP 0 793 959 A1 beschreibt Zubereitungen mit kontrollierter Wirkstoffabgabe, die mit einer wasserunlöslichen Substanz und einem quellbaren Polymer, das keine basischen Gruppen aufweist, überzogen ist. In einer langen Liste möglicher Einsatzstoffe werden bei den wasserunlöslichen Substanzen auch Polymethacrylatcopolymere mit quatemären Ammoniumgruppen (EUDRAGIT® RS) genannt. Unter den quellbaren Substanzen ist Na-Carboxymethylcellulose mit aufgeführt. Ein konkreter Hinweis diese beide Polymere zu kombinieren ist nicht enthalten.

INTERNATIONAL JOURNAL OF PHARMACEUTICS, Bd. 68, 1991, Seiten R1-R3 offenbart eine als Matrix für feste orale Arzneiformen dienende Pulvermischung, die aus Eudragit® RS und Natrium Carboxymethylcellulose besteht, jedoch kein Wasser enthält.

WO 97 41839 A lehrt die Verwendung von wässrigen Dispersionen von Eudragit® RS zur Geschmacksmaskierung.

### Aufgabe und Lösung

Die erfindungsgemäße Aufgabe wird darin gesehen, ein Überzugs- und Bindemittel für feste orale Arzneiformen zu bereitzustellen, das einerseits Wirkstoffe aus Arzneiformen schnell und ohne Einfluß des pH-Wertes freisetzt und andererseits eine gute Isolierwirkung bereits in dünnen Schichten, sowie ein hohes Pigmentbindevermögen und eine zuverlässige Geschmacksisolierung ermöglicht. Weiterhin soll die Verarbeitung in wäßrigen Medien nicht durch Quellung und Verklebungen behindert sein. Die Aufgabe wurde gelöst durch eine wäßrige Dispersion, geeignet zur Herstellung von Bindemitteln oder Überzügen für feste orale Arzneiformen, mit einem Wassergehalt von 90 bis 40 Gew.-% und einem Feststoffanteil von 10 bis 60 Gew.-%, wobei sich der Feststoffanteil aus 10 bis 99 Gew.-% einer Polymermischung A) aus
(a) 75 - 99 Gew.-% eines Polymethacrylat-Copolymers bestehend zu 98 - 85 Gew.-% aus Alkyl(meth)acrylat-Monomeren mit C₁- bis C₄-Alkylresten und zu 2 - 15 Gew.-% aus Alkyl(meth)acrylat-Monomeren mit einer quaternären Ammoniumgruppe im Alkylrest und
(b) 25 - 1 Gew.-% eines Alkalisalzes der Carboxymethylcellulose mit einem Molekulargewicht (Gewichtsmittel) unter 150.000 und
B) 90 bis 1 Gew.-% üblicher Zuschlagstoffe für Arzneimittelformulierungen zusammensetzt.

Entscheidend für die Eigenschaften der aus der Dispersion herstellbaren Überzugs- und Bindemittel für feste orale Arzneiformen ist die Polymermischung A) aus den Komponenten (a) und (b) im angegebenen Mengenverhältnis. Dabei ist es unerläßlich, daß beide Komponenten in wäßriger Umgebung vorliegen, da die vorteilhaften Effekte unter Anwendung einer organischer Lösung nicht erreicht werden können (siehe das Vergleichsbeispiel 19). Es wird vermutet, daß die Polymere (a) und (b) in der wäßrigen Phase entweder bereits in der Dispersion selbst oder aber während des Aufbringens der Dispersion oder während des Verdunstens des Wassers in vorteilhafter Weise miteinander aggregieren. Erstaunlicherweise kommt es mit steigender Menge (b) zu einer Zunahme der Reißfestigkeit, aber zu einer Abnahme der Reißdehnung. Die aus der Dispersion herstellbaren Bindemittel bzw Überzüge weisen eine gute mechanische Festigkeit auf (siehe Beispiel 10), zerfallen jedoch in künstlichem Magensaft in gewünschter Weise sehr rasch. Die Wirkstoffabgabe erfolgt praktisch konstant und pH-unabhängig, wobei die Zerfallszeit der Arzneimittelüberzüge bei einem Polymerauftrag von 2 mg /cm² in künstlichem Magensaft weniger als 30 Minuten beträgt. Die Geschmacksisolierung ist sehr zuverlässig (s Beispiel 9) und das Pigmentbindevermögen hoch (s. Beispiel 8).

### Ausführung der Erfindung

### Wassergehalt und Teilchengröße

Der Wassergehalt der Dispersion liegt im üblichen Bereich von 90 - 40 Gew.-%, bevorzugt bei 80 - 50 Gew.-%. Die mittlere Teilchengröße liegt im Bereich zwischen 50 und 500 nm.

### Die Polymermischungen A)

### - Komponente (a)

Ein weitgehend wasserunlöslicher Anteil an der Polymermischungen A aus (a) und (b) wird durch (Meth)acrylatcopolymere (a) gebildet, die zu 98 - 85 Gew -%, bevorzugt zu 96 bis 88 Gew.-% aus Alkyl(meth)acrylat-Monomeren mit C1- bis C4-Alkylresten und zu 2 - 15 Gew.-%, bevorzugt zu 4 bis 12 Gew.-% aus Alkyl(meth)acrylat-Monomeren mit einer quaternären Ammoniumgruppe im Alkylrest bestehen.

Geeignete Alkyl(meth)acrylat-Monomere mit C1- bis C4-Alkylresten sind Methylacrylat, Ethylacrylat, Butylacrylat, Butylmethacrylat und Methylmethacrylat. Bevorzugt sind im Copolymer 20 - 40 Gew.-%, insbesondere 25 - 35 Gew.-% Ethylacrylat und 50 - 70 Gew.-%, insbesondere 55 - 70 Gew.-% Methylmethacrylat entahlten.
Geeignete Alkyl(meth)acrylat-Monomere mit quaternären Ammoniumgruppen können z. B. der EP 0 181 515 B1 entnommen werden. Zu nennen sind z. B. Acryl- und Methacryloxy-trimethylammonium-chlorid und -methosulfat, Benzyldimethylammoniumethyl-methacrylat-chlorid, Diethylmethylammoniumethyl-acrylat und -methacrylat-methosulfat, N-Trimethylammoniumpropyl-methacrylamid-chlorid und N-Trimethylammonium-2,2-dimethyl-propyl-1-methacrylat-chlorid. Besonders bevorzugt ist 2-Trimethylammoniumethlymethacrylat-Chlorid.

Bevorzugte, der Komponente (a) entsprechendes (Meth)acrylatcopolymer mit quatemären Ammoniumgruppen, kann aus 65 oder 60 Gew.-% Methylmethacrylat, 30 Gew.-% Ethylacrylat und 5 bzw. 10 Gew.-% 2-Trimethylammoniumethlymethacrylat-Chlorid aufgebaut sein (EUDRAGIT® RS bzw. RL ).

Die Copolymere (a) werden in an sich bekannter Weise durch radikalische Substanz-, Lösungs-, Perl- oder Emulsionspolymerisation erhalten. Sie können als extrudiertes Granulat, gemahlenes bzw. sprühgetrocknetes Pulver oder als Dispersion z. B. mit 30 Gew.-% Feststoff vorliegen.

### Komponente (b)

Den wasserlöslichen Bestandteil (b) der Polymermischung A aus (a) und (b) bilden die Alkalisalze der Carboxymethylcellulose mit einem Molekulargewicht (Gewichtsmittel) unter 150.000, bevorzugt von 5.000 bis 100.000, besonders bevorzugt von 7.000 bis 70.000. Liegt das Molekulargewicht der Komponente (b) bei 150.000 oder darüber, kann es zur Verdickung des Ansatzes kommen, wodurch dieser praktisch nicht mehr weiterverarbeitbar ist. Geeignet sind die Alkalisalze des Lithium, Natrium und Kalium, sowie Ammonium-Salze. Bevorzugt werden die Natrium-Salze verwendet. Die Viscosität einer 2%-iger Lösung in Wasser bei 20 °C liegt meist im Bereich von 1 - 200 mPas, bevorzugt bei 2 - 60 mPas. Bevorzugt werden solche Typen eingesetzt, die auf Grund ihres Herstellungsverfahrens nur geringe Anteile an nativen Fasern enthalten.

### Mischungverhältnis der Komponenten (a) und (b)

Der Anteil der Komponente (b) beträgt 1 - 25 Gew -%, bevorzugt 5%- 15, besonders bevorzugt 5 - 10 Gew.-%, bezogen auf das nicht wasserlösliche Polymethacrylat.

Die erfindungsgemäßen Dispersionen können durch das Vermischen der Komponenten (a) und (b) in Pulverform (s. Beispiel 13) oder im Schmelzezustand (s. Beispiel 14) und jeweils anschließende Aufnahme in Wasser erfolgen. Auch können beide Komponenten bereits als Dispersion bzw. wäßrige Lösung vorliegen und direkt gemischt werden (s. Beispiel 15). Denkbar ist auch eine Extrusion der Pulvermischung mit anschließender Vermahlung zur Sicherung der gleichmäßigen Verteilung beider Komponenten und anschließende Aufnahme in Wasser.

Die Verfilmung erfolgt durch Trocknung bevorzugt während des Sprühauftrags. Der notwendige Energieeintrag für das Verdampfen des Wassers kann durch die beheizte Prozeßluft, Mikrowellen oder andere Strahlung, ggf. auch im Vakuum erfolgen.

Grundsätzlich müssen die eingesetzten Polymere toxikologisch unbedenklich und in Arzneimitteln ohne Risiko für Patienten zu verwenden sein.

### B) Übliche Zuschlagstoffe

Das erfindungsgemäße Dispersion enthält übliche Zuschlagstoffe in Mengen von 90 - 1 Gew.-% bezogen auf die Polymermischung A) aus (a) und (b). Einsatzmengen und Verwendung der üblichen Zusatzstoffe in Arzmeimittelüberzügen sind dem Fachmann geläufig. Üblichen Zuschlagstoffe können z. B. Weichmacher, Antihaftmittel, Pigmente, Stabilisatoren, Antioxidantien, Netzmittel, Porenbildner, Glanzmittel, Aromastoffe oder Geschmacksmittel sein. Sie dienen vor allem als Verarbeitungshilfsmittel und sollen ein sicheres und reproduzierbares Herstellungsverfahren sowie gute Langzeitlagerstabilität gewährleisten. Sie werden den flüssigen Polymerzubereitungen vor der Verarbeitung zugesetzt und können die Permeabilität der Überzüge beeinflussen, was ggf als zusätzlicher Steuerparameter genutzt werden kann.

### - Weichmacher:

Als Weichmacher geeignete Stoffe haben in der Regel ein Molekulargewicht zwischen 100 und 2.000 und enthalten eine oder mehrere hydrophile Gruppen im Molekül, z. B. Hydroxyl-, Ester- oder Aminogruppen. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 200 bis 2.000. Bevorzugte Weichmacher sind Triethylcitrat und Acetyltriethylcitrat.
Weiterhin zu nennen sind in der Regel bei Raumtemperatur flüssige Ester wie Citrate, Phthalate, Sebacate oder Rizinusöl.
Übliche Einsatzmengen für Weichmacher in den erfindungsgemäßen Überzugs- und Bindemitteln liegen zwischen 5 und 30 Gew.-%, bezogen auf das Polymer.

### - Antihaftmittel:

Diese Substanzen, die in der Regel lipophile Eigenschaften besitzen, werden den Sprühsuspensionen zugesetzt und verhindern eine Agglomeration der Kerne während der Befilmung. Bevorzugt werden Talkum, Mg- oder Ca-Stearat, gemahlene Kieselsäure, Kaolin oder nicht ionische Emulgatoren mit einem HLB-Wert zwischen 3 und 8 eingesetzt. Übliche Einsatzmengen für Antihaftmittel in den erfindungsgemäßen Überzugs- und Bindemitteln liegen zwischen 0 und 50 Gew.-%, bezogen auf Polymer.

### - Pigmente:

Der Zusatz erfolgt nur selten in Form des löslichen Farbstoffs. In der Regel dispergiert man Aluminium- oder Eisenoxidpigmente. Titandioxid dient als Weißpigment . Übliche Einsatzmengen für Pigmente in den erfindungsgemäßen Überzugs- und Bindemitteln zwischen 20 und 60 Gew.-%, bezogen auf die Polymermischung. Wegen des hohen Pigmentbindevermögens können jedoch auch Mengen bis zu 80 Gew.-% verarbeitet werden.

Neben den Weichmachern, Antihaftmitteln und Pigmenten sind als weitere übliche und dem Fachmann geläufige Zuschlagstoffe Stabilisatoren, Antioxidantien, Netzmittel, Porenbildner, Glanzmittel, Aromen, Geschmacksmittel zu nennen.

### Verwendung als Bindemittel:

Die Verwendung als Bindemittel erfolgt durch Aufsprühen der Dispersion auf wirkstofffreie Kerne (Nonpareilles) bei gleichzeitiger Zugabe von pulverförmigen Wirkstoffen oder deren Mischungen. Weiterhin kann auch eine wirkstoffhaltige Dispersion verfilmt werden, so daß eine flächige Arzneiform entsteht.

### Eine weitere Ausführungsform ist das Aufsprühen der Dispersion gemeinsam mit darin gelösten oder suspendierten Wirkstoffen.

### Verwendung als Überzugsmittel:

### Kerne

Träger für die Überzüge sind Kapseln, Tabletten, Granulate, Pellets, Kristalle von regelmäßiger oder unregelmäßiger Form. Die Größe von Granulaten, Pellets oder Kristallen liegt zwischen 0,01 und 2,5 mm, die von Tabletten zwischen 2,5 und 30,0 mm. Kapsel bestehen aus Gelatine, Stärke oder Cellulosederivaten.
Sie enthalten in der Regel die biologisch aktive Substanz (Wirkstoff) bis zu 95 % sowie weitere pharmazeutische Hilfsstoffe bis zu 99,9 Gew.-% Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Von besonderer Bedeutung ist die Zerfallszeit der Kerne, die Freigabe des Wirkstoffs beeinflußt. Man strebt heute kurze Zerfallszeiten von unter 5, bzw. unter 10 min im Zerfallstest nach Ph. Eur. an. Längere Zerfallszeiten sind deswegen problematisch, weil zusätzliche Überzüge die Freigabe des Wirkstoffs weiter verzögern und den therapeutischen Effekt in Frage stellen können. Als Grenzwert wird heute eine Zerfallszeit von 30 min angesehen. Vorteilhaft ist dabei ein geringer Einfluß des pH-Wertes des Zerfallsmediums. Man testet daher in Wasser und künstlichem Magensaft (0,1 N HCI).

Eingearbeitete Arzneistoffe können eine ungleichmäßige Färbung oder unangenehmen, bitteren Geschmack hervorrufen. Zur Verbesserung der Akzeptanz solche Produkte bei den Patienten strebt man eine Geschmacksisolierung von mindestens 30 sec an (s. dazu die Beispiele 9 bzw. 16).

Die eingesetzten Kerne sind homogen oder haben einen schichtartigen Aufbau. Sind Gravuren in die Oberflächen eingelassen, sollen diese durch Überzüge möglichst wenig überdeckt werden.

### Überzüge:

Die erfindungsgemäß eingesetzte Schichtdicke der Polymermischungen variiert stark und hängt von dem Verarbeitungsverfahren oder der Menge an Zuschlagstoffen ab. Sie liegt zwischen 1 und 100µm, bevorzugt zwischen 10 und 50 µm. Auf üblichen Tabletten entspricht das einem Polymerauftrag von 0,5 bis 5 Gew.-%.
Die Funktion der Polymermischung in der endgültigen Arzneiform kann vielfältig sein:
- Schutz vor schädlichen Umwelteinflüssen durch Feuchte, Gase, Licht usw.
- Geruchs- oder Geschmacksisolierung,
- Kennzeichnung durch Farbe
- Mechanische Stabilisierung
- Isolierung unverträglicher Inhaltsstoffe
- Vermeidung von Haftung an den Schleimhäuten.

Vorteilhaft ist die niedrige Viskosität der Polymermischung in wäßriger Dispersion auch bei hohen Feststoffanteilen bis zu 30 %, da Gravuren auf der Oberfläche von Tabletten detailliert nachgebildet werden.

Besonders vorteilhaft ist die gute Schutz- und Isolierwirkung der erfindungsgemäßen Polymermischung bei gleichzeitig geringem Einfluß auf den Tablettenzerfall. Insbesondere im Vergleich zu einfachen Überzügen aus HPMC wird einer bei geringen Polymeraufträgen von 1 Gew.-% schon eine Geschmacksisolierung von mehr als 30 sec. erreicht. Dickere Überzüge verbessern die Geschmacksabdeckung, ohne jedoch die Zerfallszeit zu verlängern.

Ebenso vorteilhaft ist die zuverlässige Abdeckung gefärbter Kerne durch Überzüge mit hohen Pigmentanteil. Eine besondere Ausführungsform ist die Einbettung eines zweiten Wirkstoffs in den Überzug auf einen wirkstoffhaltigen Kern.

### Auftrag des Filmüberzuges:

Auftragsverfahren erfolgt durch Gießen, Ausstreichen oder mittels Sprühauftrag aus wäßrigen Dispersionen, Suspensionen, Schmelzen oder durch direkten Pulverauftrag. Für die Ausführung ist dabei entscheidend; daß gleichmäßige, geschlossene Überzüge entstehen.

Auftragsverfahren gemäß Stand der Technik s. z. B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation relevante Eigenschaften, geforderte Tests und Spezifikationen sind in Arzneibüchern aufgelistet.

Details sind den gängigen Lehrbüchern zu entnehmen, z. B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626- 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Biologisch aktive Substanzen (bzw. pharmazeutische Wirkstoffe):

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2 die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4 Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.
Erfindungsgemäß können alle Wirkstoffe eingesetzt werden, die gewünschte therapeutische Wirkung im Sinne der obigen Definition erfüllen und eine ausreichende thermische Stabilität besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

Bevorzugte Wirkstoffe für verzögerte Wirkstoffabgabe sind: Nifedipin, Diltiazem, Theophyllin, Diclofenac Na, Ketoprofen, Ibuprofen, Indometazin, Ambroxol, Terbutalin, Vincamin, Propranolol, Pentoxyphyllin, Kodein, Morphin, Etilefrin, Carbamazepin bzw. deren therapeutische eingesetzte Salze.

### Applikationsformen:

Grundsätzlich können die beschriebenen Arzneiformen direkt durch orale Applikation zur Anwendung kommen. Die erfindungsgemäß hergestellten Granulate, Pellets, oder Partikeln können in Gelatinekapseln, Beutel (Sachets) oder in geeignete Mehrdosenbehälter mit Dosiereinrichtung abgefüllt werden. Die Einnahme erfolgt in fester Form oder suspendiert in Flüssigkeiten.

Durch Verpressen erhält man aus, ggf. nach Zumischung weiterer Hilfsstoffe, Tabletten die nach der Einnahme zerfallen und die meist überzogenen Untereinheiten freisetzen. Denkbar ist ebenso die Einbettung von Agglomeraten in Polyethylenglykol oder Lipide zur Herstellung von Suppositorien oder vaginalen Arzneiformen.

Überzogene Tabletten werden Blister oder Mehrdosenbehälter verpackt und vom Patienten direkt vor der Einnahme entnommen.

### Verwendungen

Die erfindungsgemäßen wäßrigen Dispersionen können zur Herstellung von Überzugs- und Bindemitteln für wirkstoffhaltigen Kautabletten, flächige Arzneiformen und durch Sprühtrocknung erhältliche redispergierbare Pulver verwendet werden.

### Redispergierbares Pulver

Die Erfindung betrifft somit auch ein redispergierbares Pulver erhältlich durch Sprühtrocknung einer Dispersion.

### Beispiele:

Die Beispiele 1 - 24 sind in der folgenden Übersichtstabelle stichwortartig zusammengefaßt; (a) und (b) entsprechen (a) und (b) in Anspruch 1. Die Beispiele 16 bis 20 sind Vergleichsbeipiele (nicht erfindungsgemäß).

In der Tabelle verwendete Abkürzungen:
PM1 = Polymethacrylat-Copolymer aus 10 Gew.-% Trimethylammoniumethlymethacrylat-Chlorid, 60 Gew.-% Methylmethacrylat und 30 Gew.-% Ethylacrylat
PM2 = Copolymer aus 5 Gew.-% Trimethylammoniumethlymethacrylat-Chlorid, 65 Gew.-% Methylmethacrylat und 30 Gew.-% Ethylacrylat
PM3 = Copolymer aus 30 Gew.-% Ethylacrylat und 70 Gew.-% Methylmethacrylat.
Na-CMC Typ 1 = Natrium-Carboxymethylcellulose mit einem Molekulargewicht (Mw) von ca. 50.000, Substitutionsgrad: 0,65 - 0,90, Alkaligehalt (%) Na: 7,0 - 8,9, Viskosität 2 %-ig in Wasser bei 20 °C: 25 - 50 mPas.
Na-CMC Typ 2 = Natrium-Carboxymethylcellulose mit einem Molekulargewicht (Mw) von ca. 20.000. Substitutionsgrad ca. 0,7, Alkaligehalt (%) Na: 7,0, Viskosität 2 %-ig in Wasser bei 20 °C: 2 - 3 mPas.
Na-CMC Typ 3 = Natrium-Carboxymethylcellulose mit einem Molekulargewicht (Mw) von ca. 65.000. Substitutionsgrad ca. 0,6 - 0,8, Alkaligehalt (%) Na: < 9,7, Viskosität 2 %-ig in Wasser bei 20 °C: 25 - 32 mPas.
Na-CMC Typ 4 = Natrium-Carboxymethylcellulose mit einem Molekulargewicht (Mw) von ca 250.000. Substitutionsgrad ca. 0,65 - 0,9, Alkaligehalt (%) Na: 7.0 - 8,9, Viskosität 2 %-ig in Wasser bei 25 °C: 400 - 600 mPas.
HPMC = Hydroxypropylcellulose (Methocel® E 5)

| Übersichtstabelle der Beispiele | | | |
|---|---|---|---|
| Bsp. | (a) | (b) | Stichwort |
| 1 | PM 1 | 5 % Na-CMC Typ 1 | Zerfallszeit < 20 min |
| 2 | PM 1 | 7 % Na-CMC Typ 1 | Zerfallszeit < 20 min |
| 3 | PM 1 | 15 % Na-CMC Typ 1 | Zerfallszeit < 20 min |
| 4 | PM 1 | 20 % Na-CMC Typ 1 | Zerfallszeit < 20 min |
| 5 | PM 1 | 5 % Na-CMC Typ 2 | Zerfallszeit < 20 min |
| 6 | PM 1 | 5 % Na-CMC Typ 3 | Zerfallszeit < 20 min |
| 7 | PM 2 | 10 % Na-CMC Typ 1 | Zerfallszeit < 20 min |
| 8 | PM 1 | 20 % Na-CMC Typ 1 | Pigmentbindevermögen |
| 9 | PM 1 | 9,7% Na-CMC Typ 1 | Geschmacksisolierung |
| 10 | PM 1 | 9,7 % Na-CMC Typ 1 | Abrieb |
| 11 | PM 1 | 10 % Na-CMC Typ 1 | Partikelüberzug |
| 12 | PM 1 | 8,3 % Na-CMC Typ 1 | Freigabeverhalten |
| 13 | PM 1 | 10 % Na-CMC Typ 1 | Sprühtrocknung |
| 14 | PM 1 | 10 % Na-CMC Typ 1 | Herstellung in der Schmelze |
| 15 | PM 1 | 10 % Na-CMC Typ 1 | Herstellung in Suspension |
| 16 | - | 100 % HPMC | Geschmacksisolierung |
| 17 | PM1 | 11 % Xanthan | Zerfallszeit > 30 min |
| 18 | PM3 | 10 % Na-CMC Typ 1 | Zerfallszeit > 30 min |
| 19 | PM2 | 10 % Na-CMC Typ 1 | Organische Lösung/Zerfallszeit |
| 20 | PM1 | 11 % Na-CMC Typ 4 | Na-CMC Mw= 250.000 |
| 21 | PM 1 | 10% Na-CMC Typ 1 | (aus Bsp. 11) Kautabletten |
| 22 | PM 1 | 10 % Na-CMC Typ 1 | Freigabeverhalten |
| 23 | PM 1 | 10 % Na-CMC Typ 1 | (aus Bsp. 13) Zerfallszeit |
| 24 | PM 2 | 10 % Na-CMC Typ 1 | Filmbildung |

In den Beispielen verwendete Tabletten

| Inhaltsstoffe | I Placebo-Tabletten | II Placebo-Tabletten | III Chinidinsulfat-Tabletten | IV Placebo-Tabletten mit Gravur | V Tabletten | VI Hartgelatine - kapsel |
|---|---|---|---|---|---|---|
| Cellactose® | 94,5 % | 93,3 % | 92,5 % | 94,5 % | | |
| Avicel® PH 102 | 5,0 % | 6,2 % | 5,0 % | 5,0 % | | |
| Mg-Stearat | 0,5 % | 0,5 % | 0,5 % | 0,5 % | | |
| Chinidinsulfat | | | 2,0 % | | | |
| Wirkstoff | | | | | ca. 90 % | |
| Zerfallsbeschleuniger, Formentrennmittel | | | | | ca. 10 % | |
| Acetylsalicylsäure | | | | | | 400 mg |

| **Galenische Daten der Tabletten** | | | | | | |
|---|---|---|---|---|---|---|
| | I Placebo-Tabletten | II Placebo-Tabletten | III Chinidinsulfat -Tabletten | IV Placebo-Tabletten mit Gravur | V Tabletten | VI Hartgelatine-kapseln |
| Aussehen | weiß | weiß | Weiß | weiß | dunkelbraun | Rot / grün |
| Durchmesser | 10,0 mm | 10,0 mm | 10,03 mm | 10,0 mm | 12 mm | 6,3 mm |
| Höhe | 4,0 mm | 4,1 mm | 3,91 mm | 3,59 mm | 5,5 mm | |
| Länge | | | | | | 19,6 mm |
| Gewicht | 299,3 mg (291 - 306 mg) | 306,3 mg (294 - 311 mg) | 304,6 mg (398 - 312 mg) | 310 mg | ∼ 600 mg | 487 mg |
| Härte | 52 - 74 N | 53 - 75 N | 120,4 N (113 - 133 N) | 119 N | 60 - 70 N | |
| Zerfall in entmineralisiertem Wasser | 10 - 20 min | 4 - 14 min | 13 - 20 min | ∼ 12 min | 24 - 28 min | 2 - 4 min |
| Zerfall in 0,1N HCl | 6 - 12 min. | 4 - 8 min | 14 - 20 min | ∼ 14 min | | 2 - 4 min |

### 1. Schutzüberzug auf Tabletten: (5 % Na-CMC Typ 1)

In einem Becherglas werden 9 g Talkum, 4 g Triethylcitrat und 0,9 g Na-CMC Typ 1 mit 86 g Wasser gemischt und mittels eines Dispergators Ultra Turrax T 50 (Fa. Jahnke und Kunkel) gleichmäßig dispergiert. Anschließend gießt man die Suspension langsam in 60 g einer 30%-igen Dispersion von PM1 (Eudragit® RL 30 D). das in einem Edelstahlgefäß vorgelegt wurde und mischt unter gleichmäßigem Rühren. Diese Sprühsuspension wird in einem Dragierkessel mittels einer Sprühpistole (Sprühdruck 0,8 bar) auf 1000 g Tabletten gemäß I der Tabelle über einen Zeitraum von 23 min aufgetragen. Der Gesamtauftrag beträgt 2,0 mg Polymer aus EUDRAGIT RL 30 D pro cm² Tablettenoberfläche. Während des Auftragsverfahrens wurden Teilmengen mit 1 und 1,5 mg Polymerauftrag/ cm² Tablettenoberfache entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 13 - 15 min | 14 - 18 min | 15 - 18 min |
| 0,1 N HCl. | 14 - 18 min | 15 - 19 min | 14 - 20 min |

### 2. Schutzüberzug auf Tabletten: (10 % Na-CMC Typ 1)

In einem Becherglas werden 14 Talkum, 6 g Triethylcitrat und 2,8 g Na-CMC Typ 1 mit 139 g Wasser gemischt und mittels eines Dispergators Ultra Turrax T 50 (Fa. Jahnke und Kunkel) gleichmäßig dispergiert. Anschließend gießt man die Suspension langsam in 93 g einer 30%-igen Dispersion von Polymer PM1, das in einem Edelstahlgefäß vorgelegt wurde und mischt unter gleichmäßigem Rühren. Diese Sprühsuspension wird in einem Dragierkessel mittels einer Sprühpistole (Sprühdruck 0,8 bar) auf 1500 g Tabletten gemäß II der Tabelle über einen Zeitraum von 42 min aufgetragen. Der Gesamtauftrag beträgt 2,0 mg Polymer PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet. Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 5 - 10 min | 4 - 10 min | 6 - 8 min |
| 0,1 N HCl. | 4 - 8 min | 4 - 7 min | 5 - 8 min |

### 3. Schutzüberzug auf Tabletten: (15 % Na-CMC Typ 1)

In einem Becherglas werden 9 g Talkum, 4 g Triethylcitrat und 2,7 g Na-CMC Typ 1 mit 94 g Wasser gemischt und mittels eines Dispergators Ultra Turrax T 50 (Fa. Jahnke und Kunkel) gleichmäßig dispergiert. Anschließend gießt man die Suspension langsam in 60 g einer 30%-igen Dispersion von Polymer PM1, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,8 bar) auf 1000 g Tabletten gemäß I der Tabelle über einen Zeitraum von 33 min aufgetragen. Der Gesamtauftrag beträgt 2,0 mg Polymer PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 15 - 19 min | 16 - 20 min | 15 - 19 min |
| 0,1 N HCl. | 14 - 19 min | 13 - 19 min | 14 - 21 min |

### 4. Schutzüberzug auf Tabletten: (20 % Na-CMC Typ 1)

In einem Becherglas werden 9 g Talkum, 4 g Triethylcitrat und 3,6 g Na-CMC Typ 1 mit 98 g Wasser gemischt und mittels eines Dispergators Ultra Turrax T 50 (Fa. Jahnke und Kunkel) gleichmäßig dispergiert. Anschließend gießt man die Suspension langsam in 60 g einer 30%-igen Dispersion von Polymer PM1, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,8 bar) auf 1000 g Tabletten gemäß I der Tabelle über einen Zeitraum von 33 min aufgetragen. Der Gesamtauftrag beträgt 2,0 mg Polymer PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 12 - 18 min | 14 - 19 min | 15 - 22 min |
| 0,1 N HCl. | 11 - 17 min | 12 - 16 min | 13 - 19 min |

### 5. Schutzüberzug auf Tabletten: (Na-CMC Typ 2)

In einem Becherglas werden 9 g Talkum, 4 g Triethylcitrat und 2 g Na-CMC Typ 2 mit 90 g Wasser gemischt und mittels eines Dispergators Ultra Turrax T 50 (Fa. Jahnke und Kunkel) gleichmäßig dispergiert. Anschließend gießt man die Suspension langsam in 60 g einer 30%-igen Dispersion von Polymer PM1, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,8 bar) auf 1000 g Tabletten gemäß I der Tabelle über einen Zeitraum von 30 min aufgetragen. Der Gesamtauftrag beträgt 2,0 mg PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 16 min | 17 min | 18 min |
| 0,1 N HCl. | 16 min | 16 min | 19 min |

### 6. Schutzüberzug auf Tabletten: (Na-CMC Typ 3)

In einem Becherglas werden 13,8 g Talkum, 5,5 g Triethylcitrat und 2,8 g Na-CMC Typ 3 mit 134,2 g Wasser gemischt und mittels eines Dispergators Ultra Turrax T 50 (Fa. Jahnke und Kunkel) gleichmäßig dispergiert. Anschließend gießt man die Suspension langsam in 92 g einer 30%-igen Dispersion von Polymer PM1, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,8 bar) auf 1500 g Tabletten gemäß I der Tabelle über einen Zeitraum von 27 min aufgetragen. Der Gesamtauftrag beträgt 2,0 mg Polymer pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 15 - 17 min | 14 - 17 min | 16 - 20 min |
| 0,1 N HCl. | 15 - 19 min | 18 - 20 min | 17 - 24 min |

### 7. Schutzüberzug auf Tabletten:

In einem Becherglas werden 9 g Talkum, 4 g Triethylcitrat und 1,8 g Na-CMC Typ 1 mit 90 g Wasser gemischt und mittels eines Dispergators Ultra Turrax T 50 (Fa, Jahnke und Kunkel) gleichmäßig dispergiert. Anschließend gießt man die Suspension langsam in 60 g einer 30%-igen Dispersion von Polymer PM2, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,9 bar) auf 1000 g Tabletten gemäß I der Tabelle über einen Zeitraum von 26 min aufgetragen. Der Gesamtauftrag beträgt 2,0 mg Polymer PM2 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 16 min | 17 min | 17 min |
| 0,1 N HCl. | 18 min | 18 min | 19 min |

### 8. Farbiger Überzug auf Tabletten:

In einem Becherglas werden 66 g Talkum, 5 g Triethylcitrat, 102 g einer 33%igen Polyethylenglykol 6000-Lösung in Wasser, 118 g Titandioxid und 3 g Chinolingelblack
E 104 mit 548 g Wasser gemischt und mittels eines Dispergators Ultra Turrax T 50 (Fa. Jahnke und Kunkel) gleichmäßig dispergiert. 83 g einer 30%-igen Dispersion von Polymer PM1 werden in einem Edelstahlgefäß vorgelegt, unter gleichmäßigem Rühren werden 3 g einer 33%igen Tween 80-Lösung in Wasser und 100 g einer 5%igen Na-CMC Typ 1-Lösung in Wasser zugegeben. Zu dieser Mischung werden unter Rühren die dispergierten Hilfsstoffe gegeben.
Diese Sprühsuspension wird in einem Dragierkessel mittels einer Sprühpistole (Sprühdruck 1 bar) auf 2500 g dunkel gefärbte Tabletten gemäß V der Tabelle über einen Zeitraum von 81 min aufgetragen. Der Gesamtauftrag beträgt 1,4 mg Polymer PM1 pro cm² Tablettenoberfläche. Alle überzogenen Tabletten wurden über 17 Stunden bei 40 °C getrocknet.
Die Tabletten zeigen eine glatte, gleichmäßig gelb gefärbte Oberfläche und zerfallen im Zerfallstest nach Ph. Eur in gereinigtem Wasser nach 22 bis 35 min.

### 9. Geschmacksisolierung

In einem Becherglas werden 21 g Talkum und 8 g Triethylcitrat mit 128 g Wasser gemischt und mittels eines Dispergators Ultra Turrax T 50 (Fa. Jahnke und Kunkel) gleichmäßig dispergiert. Anschließend gießt man die Suspension langsam in eine Mischung aus 137 g einer 30%-igen Dispersion von Polymer PM1, 6 g einer 33%igen Tween 80-Lösung in Wasser und 80 g einer 5%igen Na-CMC Typ 1-Lösung in Wasser, die in einem Edelstahlgefäß vorgelegt wurde, und mischt unter gleichmäßigem Rühren. Diese Sprühsuspension wird in einem Dragierkessel mittels einer Sprühpistole (Sprühdruck 0,8 bar) auf 1500 g Tabletten gemäß III der Tabelle über einen Zeitraum von 72 min aufgetragen. Der Gesamtauftrag beträgt 3,0 mg Polymer aus PM1pro cm² Tablettenoberfläche. Während des Auftragsverfahrens wurden Teilmengen mit 1,0, 1,5, 2,0 und 2,5 mg Polymerauftrag/ cm² Tablettenoberfäche entnommen. Alle überzogenen Tabletten wurden über 24 Stunden bei 40 °C getrocknet. Die Tabletten mit einem Polymerauftrag von 3 mg/cm² zerfallen im Zerfallstest nach Ph. Eur in gereinigtem Wasser und künstlichem Magensaft nach 18 bis 30 min.

Die Geschmackskaschierung des bitter schmeckenden Wirkstoffes in den Tabletten wurde durch 4 Probanden ermittelt (Mittelwerte):

| | Polymerauftrag: | 1,0 mg | 1,5 mg | 2,0 mg | 2,5 mg |
|---|---|---|---|---|---|
| | unüberzogen | | | | |
| Geschmacksisolierung (Sek.) | 0 | 55 | 127 | 173 | > 180 |

### 10. Stabilisierung der Oberfläche (Abrieb)

In einem Becherglas werden 21 g Talkum und 8 g Triethylcitrat mit 128 g Wasser gemischt und mittels eines Dispergators Ultra Turrax T 50 (Fa. Jahnke und Kunkel) gleichmäßig dispergiert. Anschließend gießt man die Suspension langsam in eine Mischung aus 137 g einer 30%-igen Dispersion von PM1, 6 g einer 33%igen Tween 80-Lösung in Wasser und 80 g einer 5%igen Na-CMC Typ 1-Lösung in Wasser, die in einem Edelstahlgefäß vorgelegt wurde, und mischt unter gleichmäßigem Rühren.
Diese Sprühsuspension wird in einem Dragierkessel mittels einer Sprühpistole (Sprühdruck 0,8 bar) auf 1500 g Tabletten gemäß III der Tabelle über einen Zeitraum von 72 min aufgetragen. Der Gesamtauftrag beträgt 3,0 mg Polymer pro cm² Tablettenoberfläche. Die überzogenen Tabletten wurden über 24 Stunden bei 40 °C getrocknet. Die Tabletten zeigen im Friabilitätstest nach USP 23 folgende Werte:

| | Friabilität |
|---|---|
| Unüberzogen | 0,04% |
| Überzogen | 0,02% |

### 11. Schutzüberzug auf Partikeln im Wirbelschichtgerät

In einem Becherglas werden 25 g Talkum und 10 Triethylcitrat mit 61 g Wasser gemischt und mittels eines Dispergators Ultra Turrax T 50 (Fa Jahnke und Kunkel) gleichmäßig dispergiert. Anschließend gießt man die Suspension langsam in eine Mischung aus 167 g einer 30%-igen Dispersion von Polymer PM1, 8 g einer 33%igen Tween 80-Lösung in Wasser und 100 g einer 5%igen Na-CMC Typ 1-Lösung in Wasser, die in einem Edelstahlgefäß vorgelegt wurde, und mischt unter gleichmäßigem Rühren.

Diese Sprühsuspension wird in einem Wirbelschichtgerät Glatt GPCG 1 mittels einer Sprühpistole (Sprühdruck 2 bar) auf 1000 g Kaliumchloridkristalle (Korngröße 0,3 - 1 mm) über einen Zeitraum von 63 min aufgetragen. Der Gesamtauftrag beträgt 5 % Polymer PM1. Die überzogenen Kristalle wurden über 24 Stunden bei 40 °C getrocknet. Die überzogenen Kristalle weisen eine Geschmackskaschierung von ca. 5 Sekunden auf und zeigen im Freigabetest analog USP 23 Paddlegerät in Wasser folgende Werte:

| Zeit [min]: | 10 | 20 | 30 |
|---|---|---|---|
| | | | |
| Wirkstoffkristalle | 97,4 % | 97,4 % | 97,4 % |

### 12. Überzug auf Kapseln

In einem Becherglas werden 12 g Talkum und 5 g Triethylcitrat mit 80 g Wasser gemischt und mittels eines Dispergators Ultra Turrax T 50 (Fa. Jahnke und Kunkel) gleichmäßig dispergiert. Anschließend gießt man die Suspension langsam in eine Mischung aus 80 g einer 30%-igen Dispersion von Polymer PM1, 3 g einer 33%igen Tween 80-Lösung in Wasser und 40 g einer 5%igen Na-CMC Typ 1-Lösung in Wasser, die in einem Edelstahlgefäß vorgelegt wurde, und mischt unter gleichmäßigem Rühren.
Diese Sprühsuspension wird in einem Dragierkessel mittels einer Sprühpistole (Sprühdruck 0,8 bar) auf 1000 g Hartgelatinekapseln gemäß VI der Tabelle über einen Zeitraum von 47 min aufgetragen. Der Gesamtauftrag beträgt 3,0 mg Polymer PM1 pro cm² Kapseloberfläche. Die überzogenen Kapseln wurden über 24 Stunden bei 40 °C getrocknet. Die Kapseln mit einem Polymerauftrag von 3 mg/cm² zerfallen im Zerfallstest nach Ph. Eur. in gereinigtem Wasser und künstlichem Magensaft nach 9 bis 15 min

Die Kapseln zeigen im Freigabetest nach USP 23 Drehkörbchenmethode in 0.1 N HCI folgende Werte:

| Zeit [min]: | 30 | 60 | 90 | 120 |
|---|---|---|---|---|
| Polymerauftrag [mg/cm²] | | | | |
| 1,2 | 2,8 % | 31,5 % | 39,6 % | 43,2 % |
| 1,5 | 1,6 % | 16,0 % | 21,5 % | 26,4 % |
| 2,2 | 1,9 % | 5,1 % | 9,8 % | 13,7 % |
| 3 | 1,7 % | 4,3 % | 6,7 % | 9,1 % |

### 13. Herstellung einer sprühgetrockneten Polymermischung

12154 g einer 30%-igen Dispersion von Polymer PM1 werden in einem Edelstahlbehälter vorgelegt und mit einem Elektrorührer gleichmäßig gerührt. Mittels Schlauchpumpe wird eine Mischung aus 7293 g einer 5%igen wäßrigen Lösung von Na-CMC Typ 1 und 553 g einer 33%igen wäßrigen Lösung von Tween 80 langsam zudosiert. Die entstandene Mischung wird mittels herkömmlicher Sprühtrocknungstechnologie getrocknet. Das resultierende Pulver ist freifließend und läßt sich in demineralisiertem Wasser redispergieren.

### 14. Herstellung der Polymermischung durch Schmelzen

In einem Edelstahlbehälter wurden 100 g Polymer PM1 in Pulverform, 10 g Na-CMC Typ 1 und 40 g Triethylcitrat gemischt und in einem Trockenschrank bei 120°C homogen aufgeschmolzen. Die Mischung wurde nach Abkühlen mittels einer IKA-Analysenmühle vermahlen. 23 g des Mahlgutes wurden mit 207 g Wasser vermischt und 24 Stunden bei 20°C mittels Magnetrührer gerührt. Die Suspension wurde in einer flachen Schale bei 40°C in einem Trockenschrank getrocknet Es resultierte ein homogener, zusammenhängender, elastischer Film.

### 15. Herstellung der Polymermischung durch direktes Suspendieren (Mischen) der Pulver

50 g Polymer PM1 in Pulverform und 5 g Na-CMC Typ 1 werden in 450 g Wasser mittels Magnetrührer eingerührt. Anschließend werden 10 Triethylcitrat zugegeben und weitere 60 Minuten gerührt.. Die Suspension wurde in einer flachen Schale bei 40°C in einem Trockenschrank getrocknet. Es resultierte ein homogener, zusammenhängender, elastischer Film.

### 16. (Vergleichsbeispiel): Geschmacksisolierung mit Hydroxypropylcellulose

In einem Becherglas werden 41 g Hydroxypropylcellulose (HPMC) in 200 g auf 60°C erwärmten Wasser gelöst. 2 g Talkum und 4 g Polyethylenglykol 6000 werden mit 145 g Wasser gemischt und mittels eines Dispergators Ultra Turrax T 50 (Fa. Jahnke und Kunkel) gleichmäßig dispergiert. Anschließend gießt man die Suspension langsam in die HPMC-Lösung. Diese Sprühsuspension wird in einem Dragierkessel mittels einer Sprühpistole (Sprühdruck 2,5 bar) auf 1500 g Tabletten gemäß III der Tabelle über einen Zeitraum von 98 min aufgetragen. Der Gesamtauftrag beträgt 3,0 mg HPMC pro cm² Tablettenoberfläche. Während des Auftragsverfahrens wurden Teilmengen mit 1,0, 1,5, 2,0 und 2,5 mg Polymerauftrag/ cm² Tablettenoberfäche entnommen. Alle überzogenen Tabletten wurden über 24 Stunden bei 40 °C getrocknet. Die Geschmackskaschierung des bitter schmeckenden Wirkstoffes in den Tabletten wurde durch 4 Probanden ermittelt (Mittelwerte):

| | Polymerauftrag | 1,0 mg | 1,5 mg | 2,0 mg | 2,5 mg | 3,0 mg |
|---|---|---|---|---|---|---|
| | unüberzogen | | | | | |
| Geschmacksisolierung (Sek.) | 0 | 8 | 15 | 17 | 21 | 24 |

### 17. (Vergleichsbeispiel): Überzug mit Xanthan

In einem Becherglas werden 9 g Talkum, 4 g Triethylcitrat und 400 g einer 0,5%igen wäßrigen Lösung von Xanthan Gum mit 77 g Wasser gemischt und mittels eines Dispergators Ultra Turrax T 50 (Fa. Jahnke und Kunkel) gleichmäßig dispergiert. Anschließend gießt man die Suspension langsam in 60 g einer 30%-igen Dispersion von PM1, das in einem Edelstahlgefäß vorgelegt wurde und mischt unter gleichmäßigem Rühren.

Diese Sprühsuspension wird in einem Dragierkessel mittels einer Sprühpistole (Sprühdruck 0,9 bar) auf 1000 g Tabletten gemäß I der Tabelle über einen Zeitraum von 70 min aufgetragen. Der Gesamtauftrag beträgt 2,0 mg Polymer PM 1 pro.cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 18 min | > 30 min | > 30 min |
| 0,1 N HCl. | 23 min | > 30 min | > 30 min |

### 18. (Vergleichsbeispiel): Überzug mit einer nicht erfindungsgemäßen Polymethacrylatdispersion

In einem Becherglas werden 13,8 g Talkum und 56 g einer 5%igen wäßrigen Lösung von Na-CMC Typ 1 mit 59 g Wasser gemischt und mittels eines Dispergators Ultra Turrax T 50 (Fa. Jahnke und Kunkel) gleichmäßig dispergiert. Anschließend gießt man die Suspension langsam in 92 g einer 30 %-igen wäßrigen Dispersion von Polymer PM3, das in einem Edelstahlgefäß vorgelegt wurde und mischt unter gleichmäßigem Rühren.
Diese Sprühsuspension wird in einem Dragierkessel mittels einer Sprühpistole (Sprühdruck 0,8 bar) auf 1500 g Tabletten gemäß I der Tabelle über einen Zeitraum von 60 min aufgetragen. Der Gesamtauftrag beträgt 2,0 mg Polymer aus EUDRAGIT NE 30 D pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 2 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 24 - > 30 min | 28 - > 30 min | > 30 min |
| 0,1 N HCl. | 29 - > 30 min | > 30 min | > 30 min |

### 19 (Vergleichsbeispiel): Überzug aus organischer Lösung:

In einem Becherglas werden 11 Granulat von Polymer PM1, 5,5 g Talkum, 2,2 g Triethylcitrat und 1,1 Na-CMC Typ 1 gelöst in 11,3 g Wasser mit 215,7 g Ethanol gemischt und mittels eines Dispergators Ultra Turrax T 50 (Fa. Jahnke und Kunkel) gleichmäßig dispergiert.
Diese Sprühsuspension wird in einem Dragierkessel mittels einer Sprühpistole (Sprühdruck 0,6 bar) auf 600 g Tabletten gemäß I der Tabelle über einen Zeitraum von 26 min aufgetragen. Der Gesamtauftrag beträgt 2,0 mg Polymer PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 2 Stunden bei 40 °C getrocknet. Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 17 - 26 min | 22 - 27 min | > 30 min |
| 0,1 N HCl. | 16 - 25 min | > 30 min | > 30 min |

### 20. (Vergleichsbeispiel) Na-CMC mit zu hohem Molekulargewicht

Schutzüberzug auf Tabletten: In einem Becherglas werden 9 g Talkum, 4 g Triethylcitrat und 2 g Na-CMC Typ 4 mit einem Molekulargewicht von ca. 250.000 und 90 g Wasser gemischt und mittels eines Dispergators Ultra Turrax T 50 (Fa. Jahnke und Kunkel) gleichmäßig dispergiert. Anschließend gießt man die Suspension langsam in 60 g einer 30 %-igen wäßrigen Dispersion von Polymer PM1, das in einem Edelstahlgefäß vorgelegt wurde und mischt unter gleichmäßigem Rühren.
Es resultiert eine breiartige, viskose Masse, die mit herkömmlichem Sprühauftrag nicht verarbeitet werden kann.

### 21. Kautabletten aus überzogenen Kristallen:

346 g überzogene Kaliumchlorid-Kristalle nach Beispiel 11 werden mit 75 g Avicel PH 102, 75 g Maisstärke, 2,5 Aerosil 200 und 1,5 Magnesiumstearat in einem Taumelmischer gemischt und anschließend mit einer Exzenterpresse (Typ Korsch EK 0) zu Tabletten mit folgenden galenischen Daten verpreßt: Durchmesser 10 mm, Gewicht 375 mg, Bruchfestigkeit 20 - 30 N, Zerfall in entmineralisiertem Wasser und 0,1 N HCI < 30 sec. Die resultierenden Tabletten weisen beim Zerkauen eine Geschmackskaschierung von ca. 10 Sekunden auf und zeigen im Freigabetest analog USP 23 Paddlegerät in Wasser folgende Werte:

| Zeit [min]: | 10 | 20 | 30 |
|---|---|---|---|
| | | | |
| Wirkstoffkristalle, überzogen | 97,4 % | 97,4 % | 97,4 % |
| Wirkstofftabletten | 96,7 % | 96,7 % | 96,7 % |

### 22. Schnellzerfallende Wirkstofftabletten aus Feuchtgranulat

1000 g Triamteren-Pulver und 1000 g Lactose EPD 80 wurden in einem Zwangsmischer Stephan UM 12 vorgelegt und mit einer Mischung aus 333 g einer 30 %-igen Dispersion von Polymer PM1 und 10 Na-CMC Typ 1 gelöst in 190 g Wasser bei 2000 Upm granuliert. Diese Mischung wurde anschließend 24 Stunden bei 40°C in einem Trockenschrank getrocknet und durch ein 1,0 mm-Sieb passiert. 2000 g des so hergestellten Wirkstoffgranulates wurden mit 20 g Crosspovidone XL und 10 g Magnesiumstearat in einem Doppelkonusmischer vermischt und auf einer Excenterpresse Korsch EK 0 bei 10 kN Preßkraft zu Tabletten mit 10 mm Durchmesser und 306 mg Gewicht verpreßt. Die so hergestellten Tabletten zeigen im Freigabetest analog USP 23 Paddlegerät in 0,1 N HCl folgende Werte:

| Zeit [min]: | 15 | 30 | 45 | 60 |
|---|---|---|---|---|
| | | | | |
| Wirkstoffgranulat | 72,2 % | 87,7 % | 94,0 % | 96,8 % |
| Wirkstofftabletten | 49,1 % | 83,7 % | 95,5 % | 98,8 % |

### 23 Überzug auf Tabletten aus redispergiertem Pulver

In einem Becherglas dispergiert man 100 g sprühgetrocknetes Produkt aus Beispiel 13 in 233 g Wasser mittels Ultra Turrax T 50 innerhalb 10 min. In einem Becherglas wurden 13,8 g Talkum und 5,5 g Triethylcitrat mit 130 g Wasser gemischt und mittels eines Dispergators Ultra Turrax T 50 (Fa. Jahnke und Kunkel) gleichmäßig dispergiert. Anschließend gießt man die Suspension langsam in 106,1 g der zuvor hergestellten Redispersion, die in einem Edelstahlgefäß vorgelegt wurde und mischt unter gleichmäßigem Rühren.

Diese Sprühsuspension wird in einem Dragierkessel mittels einer Sprühpistole (Sprühdruck 0,8 bar) auf 1500 g Tabletten gemäß I der Tabelle über einen Zeitraum von 60 min aufgetragen. Der Gesamtauftrag beträgt 2,0 mg Polymer PM1 pro cm² Tablettenoberfläche. Während des Auftragsverfahrens wurden Teilmengen mit 1 und 1,5 mg Polymerauftrag/ cm² Tablettenoberfäche entnommen. Alle überzogenen Tabletten wurden über 24 Stunden bei 40 °C getrocknet.
Die überzogenen Tabletten zeigen eine glatte und gleichmäßige Oberfläche und zerfallen im Zerfallstest nach Ph. Eur in gereinigtem Wasser und künstlichem Magensaft nach folgenden Zeiten:

| | Polymerauftrag | | |
|---|---|---|---|
| Medium | 1 mg | 1,5 mg | 2 mg |
| Wasser entmin. | 17 - 22 min | 18 - 23 min | 18 - 21 min |
| 0,1 N HCl. | 17 - 22 min | 15 - 19 min | 14 - 20 min |

### 24 Herstellung der Polymermischung mit sprühgetrocknetem Polymer PM2

50 g Pulver aus einer sprühgetrockneten 30 %-igen wäßrigen Dispersion von Polymer PM2 werden in 200 g Wasser mittels Magnetrührer für 60 Minuten redispergiert und anschließend 5 g Na-CMC Typ 1 sowie 200 g Wasser mittels Magnetrührer eingerührt und weitere 2 Stunden gemischt. Anschließend werden 10 g Triethylcitrat zugegeben und weitere 15 Minuten gerührt.. Die Suspension wurde in einer flachen Schale bei 40°C in einem Trockenschrank getrocknet. Es resultierte ein homogener, zusammenhängender, elastischer Film.

## Patentansprüche

1. Wäßrige Dispersion, geeignet zur Herstellung von Bindemitteln oder Überzügen für feste orale Arzneiformen, mit einem Wassergehalt von 90 bis 40 Gew.-% und einem Feststoffanteil von 10 bis 60 Gew.-%, wobei sich der Feststoffanteil aus 10 bis 99 Gew.-% einer Polymermischung A) aus
(a) 75 - 99 Gew.-% eines Polymethacrylat-Copolymers bestehend zu 98 - 85 Gew.-% aus Alkyl(meth)acrylat-Monomeren mit C₁- bis C₄-Alkylresten und zu 2 - 15 Gew.-% aus Alkyl(meth)acrylat-Monomeren mit einer quaternären Ammoniumgruppe im Alkylrest und
(b) 25 - 1 Gew.-% eines Alkalisalzes der Carboxymethylcellulose mit einem Molekulargewicht (Gewichtsmittel) unter 150.000 und
B) 90 bis 1 Gew.-% üblicher Zuschlagstoffe für Arzneimittelformulierungen zusammensetzt.

2. Verwendung der Dispersion gemäß Anspruch 1 zur Herstellung schnell zerfallender Überzüge für Arzneimittel.

3. Verwendung nach Anspruch 2, **dadurch gekennzeichnet, daß** die Zerfallszeit der Arzneimittelüberzüge bei einem Polymerauftrag von 2 mg /cm² in künstlichem Magensaft weniger als 30 Minuten beträgt.

4. Verwendung der wäßrigen Dispersion nach Anspruch 1 als Bindemittel für pharmazeutische Wirkstoffe.

5. Verwendung als Bindemittel nach Anspruch 4 durch Aufsprühen der Dispersion auf wirkstofffreie Kerne bei gleichzeitiger Zugabe von pulverförmigen Wirkstoffen oder deren Mischungen.

6. Verwendung nach Anspruch 4 zur Herstellung von wirkstoffhaltigen Kautabletten.

7. Verwendung nach Anspruch 4 zur Herstellung von flächigen Arzneiformen.

8. Verwendung der wäßrigen Dispersion gemäß Anspruch 1 zur Herstellung von redispergierbaren Pulvern durch Sprühtrocknung.

9. Redispergierbares Pulver erhältlich durch Sprühtrocknung einer Dispersion gemäß Anspruch 1

## Claims

1. Aqueous dispersion, suitable for the production of binding agents or coatings for solid oral drug forms, with a water content of between 90 and 40% by weight and a solid matter content of between 10 and 60% by weight, whereby the solid matter content is comprised between 10 and 99% by weight of a polymer mixture A) of
(a) 75 - 99% by weight of a polymethacrylate copolymer comprising 98- 85% by weight alkyl(meth)acrylate monomers with C₁- to C₄-aklyl radicals and 2- 15% by weight alkyl(meth)acrylate monomers with a quaternary ammonium group in the alkyl radical and
(b) 25 - 1% by weight of an alkaline salt of carboxymethylcellulose with a molecular weight (average weight) of below 150,000 and
B) 90 - 1% by weight of normal additives for drug formulations.

2. Use of the dispersion according to claim 1, for the production of rapidly disintegrating coatings for drugs.

3. Use according to claim 2, **characterised in that** the disintegration time of the drug coating for a polymer coating of 2 mg / cm² in artificial gastric juice is less than 30 minutes.

4. Use of the aqueous dispersion according to claim 1 as a binding agent for pharmaceutically active substances.

5. Use as a binding agent according to claim 4, by spraying the dispersion onto active substance-free cores with simultaneous addition of powdered active substances or mixtures thereof.

6. Use according to claim 4 for the production of active substance-containing chewable tablets.

7. Use according to claim 4, for the production of flat drug forms.

8. Use of the aqueous dispersion according to claim 1, for the production of re dispersed powders by spray drying.

9. Re-dispersible powder obtainable by spray drying of a dispersion according to claim 1.

## Revendications

1. Dispersion aqueuse, appropriée pour la production d'agents liants ou de revêtements pour des formes médicamenteuses orales solides, ayant une teneur en eau de 90 à 40 % en poids et une proportion de matière solide de 10 à 60 % en poids, dans laquelle la fraction de matière solide se compose de 10 à 99 % en poids d'un mélange de polymères
A) à base de
a) 75 à 99 % en poids d'un copolymère de polyméthacrylate qui consiste pour 98 - 85 % en poids, en des monomères de (méth)acrylate d'alkyle ayant des restes alkyle en C₁ à C₄ et pour de 2 à 15 % en poids en des monomères de (méth)acrylate d'alkyle ayant un reste ammonium quaternaire dans le reste alkyle et
b) de 25 à 1 % en poids d'un sel de méthalcalin de la carboxyméthylcellulose ayant un poids moléculaire (poids moyen) inférieur à 15.000 et
B) de 90 à 1 % en poids d'additifs usuels pour des formulations de médicament.

2. Utilisation de la dispersion conformément à la revendication 1, en vue de préparer des revêtements qui se désintègrent rapidement, pour médicaments.

3. Utilisation selon la revendication 2,
**caractérisée en ce que**
le temps de désintégration des revêtements de médicament pour une application de polymère de 2 mg/cm², est inférieur 30 minutes dans du suc gastrique artificiel.

4. Utilisation de la dispersion aqueuse selon la revendication 1, comme agent liant pour des principes actifs pharmaceutiques.

5. Utilisation comme agent liant selon la revendication 4, par pulvérisation de la dispersion sur des noyaux dépourvus de principe actif avec addition simultanée de principes actifs pulvérulents ou de leurs mélanges.

6. Utilisation selon la revendication 4, en vue de la production de comprimés à mâcher contenant des principes actifs.

7. Utilisation selon la revendication 4, en vue de la production de formes médicamenteuses de forme plane.

8. Utilisation de la dispersion aqueuse conformément à la revendication 1, en vue de la production de poudres que l'on peut remettre en dispersion par séchage par pulvérisation.

9. Poudre redispersible accessible par séchage par pulvérisation d'une dispersion conformément à la revendication 1.
